# EUROPEAN PATENT SPECIFICATION

(11) **EP 2 477 571 B1**
(45) Date of publication and mention of the grant of the patent: **06.05.2015**
(21) Application number: 10817969.8
(22) Date of filing: 20.09.2010
(51) Int. Cl.: A61B 18/24, A61M 5/44, A61N 5/067, A61B 18/28, A61B 18/04

(54) **HOT TIP LASER GENERATED VAPOR VEIN THERAPY DEVICE**
VENENTHERAPIEINSTRUMENT MIT DURCH LASER MIT HEISSER SPITZE ERZEUGTEM DAMPF
DISPOSITIF DE THÉRAPIE VEINEUSE À VAPEUR GÉNÉRÉE PAR UN LASER À POINTE CHAUDE

(30) Priority: 18.09.2009 US 243655 P
(43) Date of publication of application: 25.07.2012
(73) Proprietor: Veniti, Inc., Des Peres MO 63131 (US)
(72) Inventor: SAMSON, Wilfred, J., Des Peres, MO 63131 (US); TARTAGLIA, Joseph, M., Des Peres, MO 63131 (US); JACKSON, Jerome, Des Peres, MO 63131 (US); TREBOTICH, Steven, H., Des Peres, MO 63131 (US); GLAZE, Grant, Michael, Des Peres, MO 63131 (US); CHENG, Chun-chih, Des Peres, MO 63131 (US)
(74) Representative: Price, Nigel John King
(86) International application number: PCT/US2010/049485
(87) International publication number: WO 2011/035238

(56) References cited:
- EP-A2- 0 320 256
- WO-A1-97/33522
- WO-A1-2006/059793
- US-A- 4 476 512
- US-A1- 2002 177 846
- US-A1- 2003 109 869
- US-A1- 2003 109 869
- US-B1- 6 746 464

## Description

### BACKGROUND OF THE INVENTION

The human venous system of the lower limb consists essentially of the superficial venous system and the deep venous system with perforating veins connecting the two systems. The superficial system includes the great saphenous, small saphenous and the lateral saphenous systems. The deep venous system includes the anterior and posterior tibial veins which unite to form the popliteal vein, which in turn becomes the femoral vein when joined by the short saphenous vein.

The venous systems contain numerous one-way valves for facilitating blood flow back to the heart. Venous valves are usually bicuspid valves, with each cusp forming a sack or reservoir for blood which, under pressure, forces the free surfaces of the cusps together to prevent retrograde flow of the blood and allow antegrade flow to the heart. When an incompetent valve is in the flow path of retrograde flow toward the foot, the valve is unable to close because the cusps do not form a proper seal and retrograde flow of blood cannot be stopped.

Incompetence in the venous system can result from vein dilation, which causes the veins to swell with additional blood. Separation of the cusps of the venous valve at the commissure may occur as a result. The leaflets are stretched by the dilation of the vein and concomitant increase in the vein diameter which the leaflets traverse. Stretching of the leaflets of the venous valve results in redundancy which allows the leaflets to fold on themselves and leave the valve open. This is called prolapse, which can allow reflux of blood in the vein. Eventually the venous valve fails, thereby increasing the strain and pressure on the lower venous sections and overlying tissues. Two venous diseases which often involve vein dilation are varicose veins and chronic venous insufficiency.

The varicose vein condition includes dilatation and tortuosity of the superficial veins of the lower limb, resulting in unsightly protrusions or discoloration, 'heaviness' in the lower limbs, itching, pain, and ulceration. Varicose veins often involve incompetence of one or more venous valves, which allow reflux of blood from the deep venous system to the superficial venous system or reflux within the superficial system.

Current varicose vein treatments include invasive open surgical procedures such as vein stripping and occasionally vein grafting, venous valvuloplasty and the implantation of various prosthetic devices. The removal of varicose veins from the body can be a tedious, time-consuming procedure and can be a painful and slow healing process. Complications including scarring and the loss of the vein for future potential cardiac and other by-pass procedures may also result. Along with the complications and risks of invasive open surgery, varicose veins may persist or recur, particularly when the valvular problem is not corrected. Due to the long, arduous, and tedious nature of the surgical procedure, treating multiple venous sections can exceed the physical stamina of the physician, and thus render complete treatment of the varicose vein conditions impractical.

Newer, less invasive therapies to treat varicose veins include intralumenal treatments to shrink and/or create an injury to the vein wall thereby facilitating the collapse of the inner lumen. These therapies include sclerotherapy, as well as catheter, energy-based treatments such as direct laser, Radio Frequency (RF), or resistive heat (heater coil) that effectively elevate the temperature of the vein wall to cause collagen contraction, an inflammatory response and endothelial damage. Sclerotherapy, or delivery of a sclerosant directly to the vein wall, is typically not used with the larger trunk veins due to treatment complications of large migrating sclerosant boluses. Direct to tissue laser energy delivery can result in extremely high tissue temperatures which can lead to pain, bruising and thrombophlebitis. RF therapy is typically associated with lengthy treatment times, and resistive heater coil treatments can be ineffective due to inconsistent vein wall contact (especially in larger vessels). The catheter based treatments such as direct laser, resistive heater coil and RF energy delivery also typically require external vein compression to improve energy coupling to the vein wall. This is time consuming and can again lead to inconsistent results. In addition, due to the size and/or stiffness of the catheter shaft and laser fiber optics, none of these therapies are currently being used to treat tortuous surface varicosities or larger spider veins. They are currently limited in their use to large trunk veins such as the great saphenous vein (GSV). Tortuous surface varicosities are currently treated with sclerotherapy and ambulatory phlebectomy, while larger spider veins are currently only treated with sclerotherapy.

U.S. Patent Appl. Publ. No. 2002/0177846 describes a catheter-based vapor system for use, e.g., in treating varicose veins. In one embodiment, described in connection with Figure 19 of that publication, the device generates vapor in a larger diameter chamber proximal to a smaller diameter catheter and catheter outlet.

U.S. Patent No. 6,911,028 also describes a catheter-based vapor system for use in shrinking or otherwise modifying veins and other lumens. In one embodiment, electrodes of opposite polarity in a recessed bore near the distal end of the catheter generate and eject pressurized vapor into the vein or other lumen.

### SUMMARY OF THE INVENTION

According to the present invention there is provided the vapor generating device of claim 1.

Additional aspects of the invention are set out in the dependent claims.

The invention will be more fully understood by studying the embodiments described and illustrated hereinafter.

In some embodiments, the fiber optic can be a laser fiber optic or a fiber optic bundle.

In one embodiment, the fluid is delivered at a constant flow rate. In another embodiment, the fluid is delivered at a time varying rate. In yet another embodiment, the fluid is delivered at a rate dependent upon the diameter of the vessel or organ being treated.

In some embodiments, the power delivered to the electrode array is adjusted automatically by a controller. In other embodiments, the power is adjusted manually.

In one embodiment, the fluid is saline. In other embodiments, the fluid is electrically conductive. In additional embodiments, the fluid is non-electrically conductive.

### BRIEF DESCRIPTION OF THE DRAWINGS

Fig. 1 is a schematic view of a vapor therapy system.
Fig. 2 is close up view of a catheter for use in the vapor therapy system of Fig. 1.
Fig. 3 illustrates another embodiment of a vapor therapy system.
Fig. 4 illustrates how the absorption of photonic energy by water varies as a function of wavelength, with a general trend of higher absorption with higher frequency.
Fig. 5 illustrates examples of lasers with more favorable wavelengths for heating water, and thus making laser produced steam.

### DETAILED DESCRIPTION OF THE INVENTION

One embodiment of the invention provides a catheter-based vapor therapy system that generates vapor at a distal end. The distal tip of the catheter is small enough to fit within not only the GSV but also in smaller vessels within the patient's legs. For example, vapor generation catheters according to this embodiment may be made with diameters in the range of 1.3 mm to 3.3 mm (4 Fr to 10 Fr), which is useful for treating a common range of veins and/or blood vessels, including the GSV and major tributaries emanating from it, since the catheter's diameter will easily fit within these vessels. However, in other embodiments, the catheters can be larger than 3.3 mm (10 Fr) or smaller than 1.3 mm (4 Fr).

One aspect of vapor therapy systems according to this embodiment is the heat source at the distal end of the catheter that creates the liquid to vapor phase change, particularly the dimensions and efficiency of the heat source. In order to place the tip of the vapor catheter in a small lumen, such as a leg vein smaller than the GSV, a catheter diameter of 2.3 mm (7 Fr) or less may be desirable. While it might be possible to generate vapor outside the patient and deliver it via a catheter to the treatment site, generation and delivery remote from the treatment site raises issues with respect to protection of both patient and clinician from burns and with respect to the quality or latent energy of the vapor delivered. Generation of vapor at the catheter's distal outlet, however, raises issues with respect to the size and configuration of the heat source. In particular, the heat source must not only be small enough to fit within the catheter tip, it must also permit sufficient liquid and vapor flow to provide the desired therapeutic benefit. One such small but effective very heat source is a source of laser or light energy. Laser energy is an acronym for Light Amplification by the Stimulated Emission of Radiation. Sources of laser energy can be a laser diode, light emitting diode, or other device that delivers photonic (light) energy into the fluid used to generate the vapor. Vapor is generated because the fluid is heated by absorption of this photonic energy. Another benefit of using a laser instead of an energy source like radiofrequency energy is that the fluid would not necessarily need to be conductive allowing the use of sterile, de-ionized water for injection.

The hereinafter described and illustrated embodiments of fiber optic laser delivery system may be used to create and then to deliver steam to the treatment area. The delivery system may utilize a fiber optic catheter, which delivers energy to a distal fluid containing capsule that has a predetermined amount of liquid in the form of water or other liquid that is converted to steam by the application of laser energy. The capsule has a single or multiple small ports or orifices, that allow for a steam to be directed outward or forward to blood vessel walls. There may be a single port, or multiple ports for steam distribution. Energy to cause liquid vaporization is achieved by the application of laser energy delivered via a flexible fiber optic catheter directed to a liquid filled distal catheter capsule. Such a catheter has distinct advantages over systems presently in use today. First the flexible fiber optic delivery catheter shaft can remain relatively small and flexible allowing for improved vessel navigation and catheter placement. It will remain cool to touch while being energized, and hence has the advantage of pain reduction to the patient. At the distal extremity, resides a small fluid chamber which contains a vaporizable fluid. The fluid is heated by the application of laser energy applied from a fiber optic energy delivery element contained within a catheter shaft. The fiber optic element may be contained in a separate lumen, or a shared lumen. The advantage of utilizing the vaporization of a fluid, such as water, is the ability to more precisely control the temperature of the therapeutic treatment medium at the site of application. In addition to temperature control by virtue of steam utilization, one can further have the ability to control the specific volumes of steam needed for application to a particular vessel size. The catheter shaft includes a fiber optic bundle with a lumen way for continuous or intermittent replenishment of fluid to be vaporized within a capsule at the catheter distal end. Fluid introduction can be accomplished by metering a controlled amount of liquid in combination with the intermittent or continuous application of laser energy for controlled vapor generation.

Such a combination as described by this invention can result in cooler more flexible, navigable, catheter shaft, along with more precise temperature control of the therapeutic energy medium. The invention can involve measuring capsule temperature to ensure that the ideal desired vaporization temperature is not exceeded or fluid has not been depleted in the vaporization capsule. The use of thermocouples or other temperature sensing elements can also be used in conjunction with controlling either continuous or intermittent application of the applied laser energy level, also capsule temperature feedback can be used to control or regulate the introduction of intermittent or continuous flow of fluid to the distal chamber.

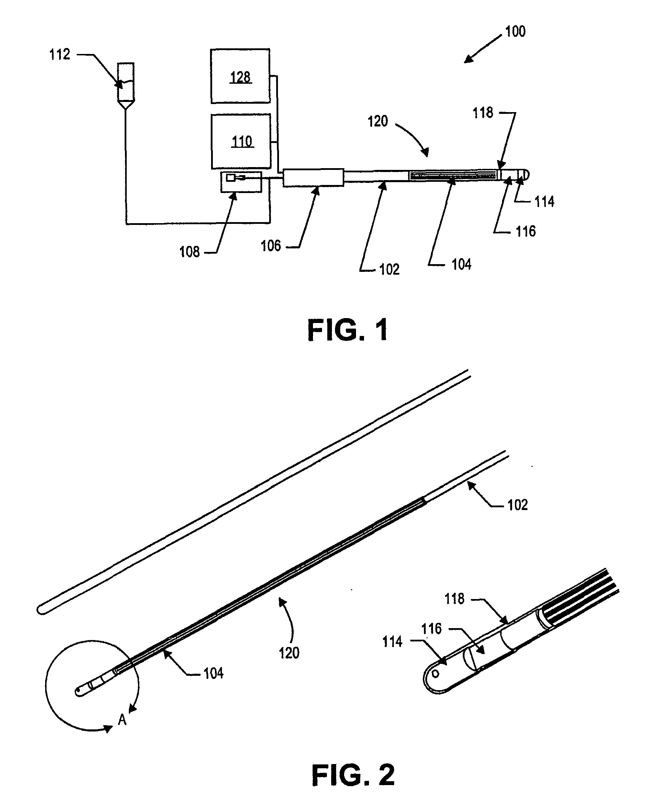

Figs. 1 and 2 illustrate a vapor therapy system 100 according to one embodiment of the invention. System 100 includes catheter 102, laser fiber optic or fiber optic bundle 104, a sensor 118, hand piece 106, pump 108, laser generator console 110, fluid reservoir 112, and vapor port(s) 114. System 100 may further include flow restrictor 116 and further sensors 118.

In this embodiment, fluid can be provided from a fluid reservoir 112 to catheter 102 by pump 108, which can be a positive displacement pump, a peristaltic pump, a syringe pump, or other fluid metering system, for example. The fluid may be, e.g., normal sterile (0.9%) saline or sterile water for injection. Other suitable fluids are those that are electrically conductive or not and compatible with the human body, and can include other concentrations of sterile saline, such as hypertonic concentrations of 1%, 2%, etc., and hypotonic concentrations of 0.8%, 0.7%, etc., fluids containing other ionic molecules, such as potassium chloride, etc. In addition to a pump, the fluid can be dispensed from a pressurized reservoir, such as a saline or water for injection bag with a pressure cuff, and be metered out precisely with a valve. The pump or other pressurized fluid source can deliver fluid to the catheter, where it enters a fluid delivery lumen that extends from a liquid inlet to a vapor generation chamber 120 within the catheter. Prior to use to shrink leg veins to treat varicose veins, the saline, water for injection or other liquid can be provided from the fluid reservoir (such as a saline drip bag) to the pump and injected into the catheter at low flow rates of, for example, 1 drop to 15 drops per minute in a stand-by mode to prevent retrograde flow of blood or other physiologic material into the catheter and to prevent clotting within or near the catheter. Alternatively, an anticoagulant, such as heparin, can be added to the fluid drip which enhances its ability to prevent clots in or near the catheter body. Liquid can drip out of the vapor ports of the vapor generation catheter in the stand-by mode.

The catheter can be adapted to be inserted into the vessels of a patient's legs. In one embodiment, the outer diameter of the catheter can be formed from polyimide or from any other suitable material, such as Pebax, Polyamide, nylon, Hytrel, or other biocompatible plastic, rubber, thermoset, thermoplastic, or elastomeric material. The catheter shaft can also be braided to increase kink resistance and column strength or pushability.

The distal tip of the catheter has one or more vapor exit ports 114 downstream of or distal to the flow restrictor. For example, the vapor generation catheter tip shown in Figs. 1 and 2 can be fabricated out of heat resistant stainless steel or equivalent material, and have a distal end with side vapor exit ports formed therein. Other vapor exit ports may be arranged around the circumference and/or on the distal end, as needed. In addition, the vapor port can be on the leading end of the catheter to allow the vapors to be dispensed forward. Some suitable vapor exit port configurations as shown, for example, in USSN 61/059,518.

In the embodiment illustrated in Fig. 1, an optional hand piece 106 can be provided at the proximal end of the catheter for convenience in placing and moving the catheter. Connections to the liquid source and to the power source may be made through the hand piece. The length of the hand piece and of the catheter itself can be modified to fit the intended application. For example, to treat veins within human legs, the length of the catheter distal to the hand piece may be between 10 cm and 100 cm, however longer lengths to accommodate longer legs can be fabricated.

As shown in Figs. 1-2, the vapor generation chamber 120 can include a laser fiber optic or fiber optic bundle 104 having a particularly small form factor. The vapor generation chamber can be disposed in a distal portion of the catheter 102 and be in fluid communication with the fluid reservoir 112 and vapor ports 114. The vapor generation chamber can also be located anywhere within the length of the catheter shaft, or even in the hand piece embodiments, any number of electrode pairs can be used.

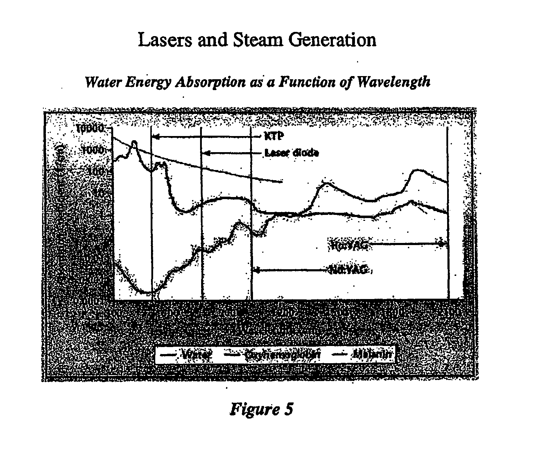

In one embodiment, the laser generator can be a solid-state diode laser with a wavelength of 1100nm. In another embodiment, wavelength could be within the range of 600 to 11600nm as seen in Fig. 5 and as described in the following paragraphs.

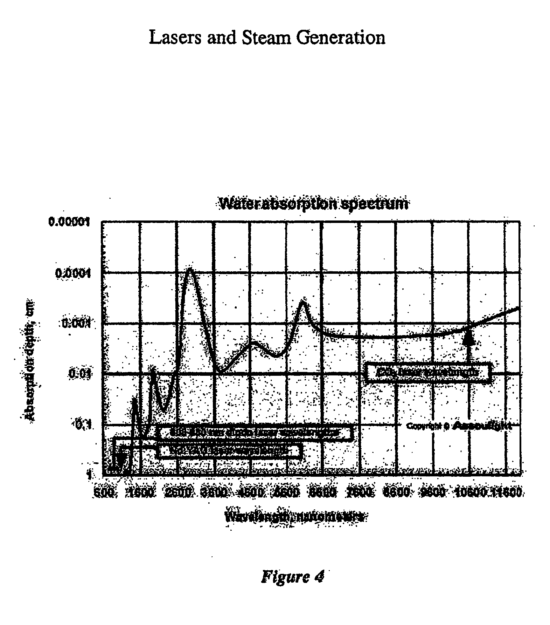

Laser energy heats a fluid such as water by absorption of photonic energy. If sufficient energy per unit mass of water is absorbed, the water will change phase from liquid to gaseous/vapor (steam). As shown in Fig. 4, the absorption of photonic energy by water varies as a function of wavelength, with a general trend of higher absorption with higher frequency. Many lasers have the ability to heat water; however the efficiency increases for sources with relatively long wavelengths. Therefore, for higher efficiency, the choice of laser type and wavelength is critical for maximizing the laser energy that can be delivered to the water molecule. As can be seen, water has favorable absorption properties in the long wavelength area, e.g. 1100 nm and above. Thus, lasers with wavelengths in this range will be the most effective source for laser heating of water and laser steam generation. As seen in Fig. 5, examples of lasers with more favorable wavelengths for heating water, and thus making laser produced steam, include near-infrared (1390 nm) and mid-infrared (5940 nm), Nd:YAG (1200 nm), InGaAsP family (1300 - 1600 nm), Hydrogen-Fluoride Chemical (2600 - 3000 nm), and CO₂ (10600 nm). Increasing the optical path length in contact with the water also helps increase absorption of photonic energy. Thus, the effect of using lower water absorption laser wavelengths can be somewhat mitigated buy increasing the optical path length to improve the ability of lower wavelength lasers to heat water and produce steam.

The system includes one or more sensors, such as temperature or pressure sensors (not shown). At least one sensor senses a temperature of the laser fiber optic. The system, such as the controller or the sensors, can monitor the flow rate of fluid delivered to the catheter, the power levels of the laser generator, and the temperature of the laser diode tip or the fluid within the vapor generation chamber. The controller adjusts the power of the laser generator, based on the sensed temperature of the laser fiber optic.

The diode laser fiber optic or fiber optic bundle can be inside the center of the vapor generation chamber 120 disposed at the outlet of the liquid supply lumen. As shown in Fig. 2, the vapor generation chamber can be positioned at a distal portion of the catheter. A flow restrictor 116 can be disposed between the vapor generation chamber and a vapor port 114. The flow restrictor can be sized to ensure that the pressure within the vapor generation chamber is high enough so that the vapor is superheated when it exits the catheter. The vapor can be in the range of 100 to 140 degrees when it exits the catheter, or higher if higher steam quality is desired. In the embodiment shown in Figs. 1 and 2, the flow restrictor is formed from porous PTFE bonded in place within the catheter using high-temperature adhesive. In other embodiments, a metal based filter material, duckbill valve, ball check valve, micro-lumens or a flow control orifice may be used as the flow restrictor.

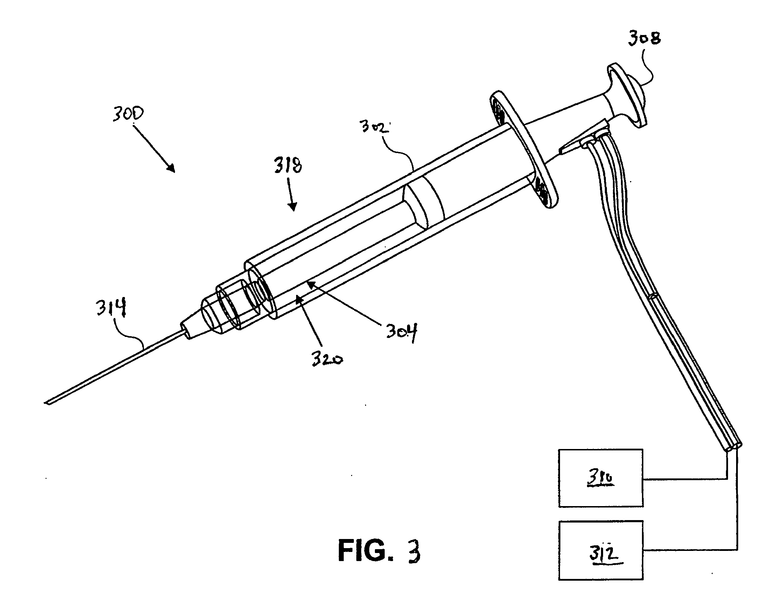

Fig. 3 illustrates a vapor therapy system 300 according to one embodiment of the invention. System 300 includes body 302, laser fiber or fiber optic bundle 304, plunger (pump) 308, laser generator 310, fluid reservoir 312, and delivery needle 314. System 300 further includes at least one sensor 318. Laser fiber optic or fiber optic bundle 304, laser generator 310, fluid reservoir 312, fluid pressurization and dispensing control system, and sensors 318 of system 300 can correspond, respectively, to laser fiber optic or fiber optic bundles 104 laser, laser generator 110, fluid reservoir 112, and sensors 118 described above. However, the vapor therapy system 300 shown in Fig. 3, with the inclusion of a vapor delivery needle 314, can be better suited for accessing and treating small superficial veins and/or surface varicosities than the system 100 of Fig. 1.

Body 302 can be a rigid or semi-rigid elongate body, and can house laser fiber optic or fiber optic bundle 304. The delivery needle 314 can be disposed on a distal end of the body and be in fluid communication with the laser fiber optic or fiber optic bundle and fluid reservoir 312. In other embodiments, the delivery needle 314 can be disposed on a vapor delivery catheter, such as catheter 102 described above. In one embodiment the output from a vapor generation chamber is diverted away from the tip to maintain vapor quality while keeping the temperature of the tip low. Plunger 308 can be advanced to open a valve located at the distal end of a chamber and allow the developed vapor to flow from a vapor generation chamber 320 of the body through needle 514 into a patient. In another embodiment, Plunger 308 can be in fluid communication with a reservoir and a laser fiber optic or fiber optic bundle can be in fluid communication with the reservoir, but the fluid is not sufficiently pressurized to cause significant flow. Plunger 308 can be advanced increasing the flow and pressure of the fluid which is heated by the laser fiber optic or fiber optic bundle to generate vapor. A switch may be incorporated in Plunger 308 that is in electrical communication with the generator such that when Plunger 308 is advanced a signal is sent to the generator to initiate energy delivery to the laser fiber optic or fiber optic bundle. Plunger 308 may be stationary and include a switch on it that is in electrical communication with the generator such that when the switch is actuated a signal is sent to the laser generator to initiate energy delivery to the laser fiber optic or fiber optic bundle. In some embodiments, the plunger is replaced with a pump, such as the pumps described above. The delivery needle 314 is typically adapted to be inserted into a vein of a patient while the body 302 is positioned outside of a patient. However, in other embodiments, both the body and needle can be positioned inside the patient.

The needle can be disposed on a distal end of the delivery lumen of the elongate member described herein, for example. Once the vapor is generated and delivered out of the tip of the needle, it can easily travel through the vein and successfully traverse the tortuosities; catheter or needle access along the entire desired treatment length need not be achieved. Thus, a single, or a reduced number of needle sticks can be required to treat an extensive network of small or tortuous superficial veins. The needle can be a standard hypodermic needle or it can include other vapor ports. The outer shaft of the needle can be insulated, either passively by incorporating a low heat conducting material onto its outer surface or by including an active cooling insulating sleeve. Such passive insulating materials may be, but not be limited to, Teflon (PTFE), polyimide, paralyne, polyethylene, polypropylene, PET, PTFE, PMMA, PVC, Mylar, Nylon or ceramic materials. The needle can be fabricated of metal such as stainless steel or it can be fabricated of a polymer or ceramic such as Teflon and others, some of which are listed in the prior sentence.

A method for generating steam in a catheter or other elongate delivery device will now be discussed with reference to Figs. 1 and 2. Referring to Fig. 1, fluid can be delivered from fluid reservoir 112 to vapor generation chamber 120 by pump 108 or other pressurized means. The fluid can be sterile saline, sterile water for injection or any other appropriate fluid. The fluid can be delivered at a constant or variable fluid flow rate. For example, in one embodiment a preferred constant flow rate is approximately 3 ml/minute. In some embodiments, controller 110 can control and adjust the fluid flow rate.

Next, the laser generator 110 can provide power to the laser fiber optic or fiber optic bundle 104 disposed in the vapor generation chamber 120. During generation of vapor, the fluid vapor generation chamber can be described with respect to three distinct regions, including warming region, steam generation region, and fully developed steam region. As the fluid flows through the vapor generation chamber and power is applied to the laser fiber optic or fiber optic bundles, the fluid is warmed in the warming region. When the fluid is heated to a sufficient temperature, such as 100 degrees Centigrade at atmospheric pressure, the fluid begins to transform into a vapor or steam in the steam generation region. All of the fluid can then be transformed into vapor by the time it reaches the fully developed steam region, after which it can exit the distal end of the vapor generation chamber and exit the catheter. If the pressure in the chamber is greater than atmospheric pressure, higher temperatures will be required and if it is lower than atmospheric pressure, lower temperatures will generate vapor.

During vapor generation, a signal corresponding to the vapor therapy system is sensed to determine if a fluid has fully developed into a vapor before exiting a distal end of the vapor generation chamber. In some embodiments, the signal is sensed by the controller 128. Sensing whether the vapor is fully developed can be particularly useful for many surgical applications, such as in the treatment of varicose veins, where delivering high quality fully developed vapor to the veins results in more effective treatment. In particular, the temperature of the laser fiber optic or fiber optic bundle is sensed.

The sensed signal can indicate the respective sizes and/or positions of the warming region, steam generation region, and fully developed steam region within the vapor generation chamber.

Thus, the power delivered to the electrode bundle is adjusted based on the sensed signal to fully develop vapor in the vapor generation region of the vapor generation chamber. This allows the vapor therapy system described herein to deliver a fully developed vapor from the catheter to a target tissue. In some embodiments of method, the power can be adjusted by controller 128. In other embodiments, the power can be adjusted by manipulation of the laser generator itself. The sensed signal is the temperature of the laser fiber optic or fiber optic bundle. For example, then the power delivered to the laser fiber optic or fiber optic bundle can be increased as the temperature decreases to ensure that vapor is fully developed before leaving the vapor generation chamber. If the temperature increases, then the power delivered to the laser fiber optic or fiber optic bundle can be decreased to maintain the ideal amount of heating area within the vapor generation region.

To provide heat therapy to the vein, the distal tip of the vapor generation catheter can be inserted into the patient's vein to place the distal tip at a desired location of vein lumen reduction. Ultrasound or fluoroscopy may be used to assist catheter placement.

Saline, water for injection or other liquid can then provided by the pump to the vapor generation catheter at a rate of 1 to 5 ml/minute, for example, and the laser generator can provide power to the laser fiber optic or fiber optic bundle. The liquid flow rate and the level of laser power provided are interdependent, and one or both may be adjusted based on vein size, vein flow, and/or a sensed signal. In addition, a thermocouple or other temperature measurement device may be provided at the distal end of the vapor generation catheter to provide feedback to the laser generator based on vapor temperature. The generator may also be controlled based on temperature as described above, to ensure that only fully developed vapor is delivered from the catheter to the vein.

As vapor condenses on venous tissue, the released latent energy or heat of vaporization causes the vein walls to shrink inward. To treat a length of vein, the distal tip of the vapor generation catheter may be drawn back as vapor is delivered, such as at a rate of about 1 cm/sec. The actual speed of pull-back depends in large part on the diameter of the vein and the volume and temperature of the steam being delivered, of course.

As for additional details pertinent to the present invention, materials and manufacturing techniques may be employed as within the level of those with skill in the relevant art. The same may hold true with respect to method-based aspects of the invention in terms of additional acts commonly or logically employed. Also, it is contemplated that any optional feature of the inventive variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this invention belongs. The breadth of the present invention is not to be limited by the subject specification, but rather only by the plain meaning of the claim terms employed.

## Claims

1. A vapor generating device (300), comprising:
a vapor generation chamber (120, 320), the vapor generation chamber having a laser fiber optic (104, 304) disposed therein;
a fluid reservoir (112, 312) coupled to the vapor generation chamber so as to deliver fluid to the vapor generation chamber; and
a laser generator (110, 310) coupled to the laser fiber optic;
**characterized in that** the device further comprises a controller (128) configured to sense a temperature of the laser fiber optic and to adjust a power delivered by the laser generator to the laser fiber optic based on the sensed temperature so as to transform all fluid delivered from the fluid reservoir to the vapor generation chamber into a vapor within the device.

2. The device of claim 1, wherein the fluid is saline.

3. The device of claim 1, further comprising a delivery needle (314) coupled to the vapor generation chamber.

4. The device of claim 1, wherein the fluid is non-electrically conductive.

5. The device of claim 1, further comprising one or more ports (114) at a distal end of the device for the outward direction therethrough of the vapor.

6. The device of claim 1, wherein the laser generator (110, 310) is a solid-state diode laser.

7. The device of claim 1, wherein the controller (128) is configured to monitor at least one of a flow rate of the fluid delivered to the vapor generation chamber (120, 320), a power level of the laser generator (110, 310), or a temperature of the fluid within the vapor generation chamber.

8. The device of claim 1, wherein the controller (128) is configured to adjust a flow rate of the fluid delivered to the vapor generation chamber (120, 320) based on the sensed temperature.

9. The device of claim 1, wherein the laser generator (110, 320) is configured to deliver energy at a wavelength between 600 nanometers and 11,600 nanometers.

10. The device of claim 1, wherein the vapor generation chamber (120, 320) includes a warming region, a steam generating region, and a fully developed steam region, and said regions are arranged so that, in use, the fluid delivered from the fluid reservoir to the vapor generation chamber will flow through the warming region, the steam generating region, and the fully developed steam region in that order.

## Patentansprüche

1. Dampferzeugungsvorrichtung (300) umfassend:
eine Dampferzeugungskammer (120, 320), wobei in der Dampferzeugungskammer eine Laser-Faseroptik (104, 304) angeordnet ist;
ein Flüssigkeitsbehälter (112, 312), der mit der Dampferzeugungskammer derart gekoppelt ist, dass der Dampferzeugungskammer Flüssigkeit zugeführt wird; und
einen Lasererzeuger (110, 310), der mit der Laser-Faseroptik gekoppelt ist;
**dadurch gekennzeichnet, dass** die Vorrichtung weiterhin eine Steuerung (128) umfasst, die ausgestaltet ist, eine Temperatur der Laser-Faseroptik zu messen und eine Leistung, die der Laser-Faseroptik durch den Lasererzeuger zugeführt wird, abhängig von der gemessenen Temperatur derart anzupassen, dass die gesamte Flüssigkeit, die der Dampferzeugungskammer von dem Flüssigkeitsbehälter zugeführt wird, innerhalb der Vorrichtung in Dampf umgewandelt wird.

2. Vorrichtung nach Anspruch 1, wobei die Flüssigkeit salzhaltig ist.

3. Vorrichtung nach Anspruch 1, weiterhin umfassend eine Zuführnadel (314), die mit der Dampferzeugungskammer gekoppelt ist.

4. Vorrichtung nach Anspruch 1, wobei die Flüssigkeit nicht elektrisch leitend ist.

5. Vorrichtung nach Anspruch 1, weiterhin umfassend einen oder mehrere Anschlüsse (114) an einem distalen Ende der Vorrichtung durch die der Dampf nach außen geführt wird.

6. Vorrichtung nach Anspruch 1, wobei der Lasererzeuger (110, 310) ein Halbleiter-Diodenlaser ist.

7. Vorrichtung nach Anspruch 1, wobei die Steuerung (128) ausgestaltet ist, mindestens eines von einer Durchflussrate der der Dampferzeugungskammer (120, 320) zugeführten Flüssigkeit, einer Leistungsstufe des Lasererzeugers (110, 310) oder einer Temperatur der Flüssigkeit innerhalb der Dampferzeugungskammer zu überwachen.

8. Vorrichtung nach Anspruch 1, wobei die Steuerung (128) ausgestaltet ist, eine Durchflussrate der der Dampferzeugungskammer (120, 320) zugeführten Flüssigkeit abhängig von der gemessenen Temperatur anzupassen.

9. Vorrichtung nach Anspruch 1, wobei der Lasererzeuger (110, 320) ausgestaltet ist, Energie in einer Wellenlänge zwischen 600 Nanometern und 11600 Nanometern zuzuführen.

10. Vorrichtung nach Anspruch 1, wobei die Dampferzeugungskammer (120, 320) einen Erwärmungsbereich, einen Dampferzeugungsbereich und einen Bereich, in dem der Dampf vollständig ausgebildet ist, beinhaltet, und wobei die Bereiche derart angeordnet sind, dass, in Betrieb, die Flüssigkeit, die der Dampferzeugungskammer von dem Flüssigkeitsbehälter zugeführt wird, den Erwärmungsbereich, den Dampferzeugungsbereich und den Bereich, in dem der Dampf vollständig ausgebildet ist, in der genannten Reihenfolge durchfließt.

## Revendications

1. Dispositif générateur de vapeur (300), comprenant :
une chambre génératrice de vapeur (120, 320), la chambre génératrice de vapeur comportant une fibre optique à laser (104, 304) disposée dans celle-ci;
un réservoir de fluide (112, 312) couplé à la chambre génératrice de vapeur de manière à délivrer un fluide à la chambre génératrice de vapeur ; et
un générateur de laser (110, 310) couplé à la fibre optique à laser ;
**caractérisé en ce que** le dispositif comporte en outre un dispositif de commande (128) conçu pour détecter une température de la fibre optique à laser et pour régler une puissance fournie par le générateur de laser à la fibre optique à laser en fonction de la température détectée de manière à transformer tout le fluide délivré à partir du réservoir de fluide à la chambre génératrice de vapeur en une vapeur à l'intérieur du dispositif.

2. Dispositif selon la revendication 1, dans lequel le fluide est une solution salée.

3. Dispositif selon la revendication 1, comprenant en outre une aiguille d'injection (314) couplée à la chambre génératrice de vapeur.

4. Dispositif selon la revendication 1, dans lequel le fluide est non conducteur de l'électricité.

5. Dispositif selon la revendication 1, comprenant en outre un ou plusieurs orifices (114) au niveau d'une extrémité distale du dispositif pour diriger à travers ceux-ci la vapeur vers l'extérieur.

6. Dispositif selon la revendication 1, dans lequel le générateur de laser (110, 310) est un laser à diode à semiconducteur.

7. Dispositif selon la revendication 1, dans lequel le dispositif de commande (128) est conçu pour contrôler au moins un élément parmi un débit du fluide délivré à la chambre génératrice de vapeur (120, 320), un niveau de puissance du générateur de laser (110, 310), ou une température du fluide à l'intérieur de la chambre génératrice de vapeur.

8. Dispositif selon la revendication 1, dans lequel le dispositif de commande (128) est conçu pour régler un débit du fluide délivré à la chambre génératrice de vapeur (120, 320) en fonction de la température détectée.

9. Dispositif selon la revendication 1, dans lequel le générateur de laser (110, 320) est conçu pour délivrer une énergie à une longueur d'onde de 600 nanomètres à 11600 nanomètres.

10. Dispositif selon la revendication 1, dans lequel la chambre génératrice de vapeur (120, 320) comporte une région de chauffage, une région génératrice de vapeur, et une région de vapeur parfaitement développée, et lesdites régions sont disposées de telle sorte que, lors de l'utilisation, le fluide délivré à partir du réservoir de fluide à la chambre génératrice de vapeur circule à travers la région de chauffage, la région génératrice de vapeur, et la région de vapeur parfaitement développée, dans cet ordre.
